(19) Europäisches Patentamt
European Patent Office
Office européen des brevets

(11) Veröffentlichungsnummer: **0 167 696 B1**

# (12) EUROPÄISCHE PATENTSCHRIFT

(45) Veröffentlichungstag der Patentschrift: **18.01.89**

(51) Int. Cl.4: **C 12 P 5/02**, C 12 M 1/00

(21) Anmeldenummer: **84890126.0**

(22) Anmeldetag: **10.07.84**

## (54) Anlage zur Herstellung von Biogas.

(43) Veröffentlichungstag der Anmeldung: **15.01.86 Patentblatt 86/3**

(45) Bekanntmachung des Hinweises auf die Patenterteilung: **18.01.89 Patentblatt 89/3**

(84) Benannte Vertragsstaaten: **BE CH DE FR GB IT LI LU NL SE**

(56) Entgegenhaltungen:
**DE-A- 3 243 103**
**FR-A- 994 032**
**FR-A- 2 440 678**
**FR-A- 2 537 992**
**US-A- 4 022 665**
**US-A- 4 204 842**

(73) Patentinhaber: **Weymelka, Walter, Eschenallee 7/2/9, A-1100 Wien (AT)**

(72) Erfinder: **Weymelka, Walter, Eschenallee 7/2/9, A-1100 Wien (AT)**

(74) Vertreter: **Itze, Peter, Dipl.-Ing. et al, Patentanwälte Dipl.-Ing. Leopold Friebel Dipl.-Ing. Peter Itze Amerlingstrasse 8, A-1060 Wien (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung bezieht sich auf eine Anlage zur Herstellung von Biogas, in welcher organische Stoffe mit Abfallmaterialien, insbesondere aus landwirtschaftlichen Betrieben, in mehreren Stufen zu Methangas vergoren werden, mit einem Behälter für die aerobe Angärung und mehreren Gärkesseln wobei die Gärkessel über eine Einrichtung zur Umwälzung des Gärmaterials verfügen.

Bisher wird jedes Material für sich behandelt und einem eigenen Methangärbehälter zugeführt. Dies hat den Nachteil, dass eine Vielzahl von Methangärbehältern notwendig ist, in denen dann die Bedingungen für die Methangärung exakt eingehalten werden müssen, um eine Störung im Stoffwechsel der methanbildenden Bakterien zu verhindern, da diese Bakterien hinsichtlich der Einhaltung ihrer Lebensbedingungen sehr anspruchsvoll sind. Ausserdem können bei diesem bekannten Verfahren nicht alle anfallenden Materialien in gleicher effizienter Weise zu Methan vergoren werden.

Weiters ist es bereits bekannt, bei reinen Methangärungen, also bei welchen sowohl die Säuerungsphase als auch die Methangärungsphase anaerob geführt wird, pflanzliche Abfallstoffe in die Säuerungsphase und kohlenstoffhaltige Gase in die Methangärung einzubringen. Bei diesem bekannten Verfahren bei welchem also keine aerobe Vorgärstufe vorgesehen ist, ist der Nachteil gegeben, dass die Säuerungsphase vielfach nicht ordnungsgemäss verläuft, so dass das in die Methangärung eingeführte Material nur ungenügend für die Methangärung aufgeschlossen ist.

Bei einer bekannten Ausbildung der eingangs genannten Art wird die Umwälzung der Biomassen durch Vorsehen ungleich temperierter Wasserkreisläufe an den Kesselwänden und der damit erzielten thermischen Zirkulation bewirkt. Eine derartige Umwälzung hat jedoch den Nachteil, dass keine ausreichende Zerstörung der Schwimmdecke erzielt und das Absetzen von Sedimenten nicht mit Sicherheit verhindert werden kann.

Der Erfindung liegt die Aufgabe zugrunde, eine Anlage der eingangs genannten Art zu schaffen, bei welcher der dargestellte Nachteil vermieden ist und welche ohne Fremdenergie betreibbar ist. Erfindungsgemäss wird diese Aufgabe dadurch gelöst, dass die Einrichtung zur Umwälzung des Gärmaterials durch wenigstens zwei, kappenartige, vorzugsweise schirmförmige, Einbaukörper gebildet ist, welche mit ihren offenen Seiten zueinander an einem Rohr angebracht sind, welches an einer zentralen Achse in seiner Längsrichtung verschiebbar geführt ist. Bei einer solchen Ausbildung sammelt sich das bei der Gärung entstehende Gas unterhalb des oberen Einbaukörpers, wodurch der Auftrieb der Umwälzeinrichtung so gross wird, dass sie sich in der Flüssigkeit aufwärtsbewegt, u.zw. so weit, bis sich der obere Einbaukörper oberhalb des Flüssigkeitsspiegels befindet, so dass dann das unterhalb des oberen Einsatzkörpers befindliche Gas in den Gasraum oberhalb des Flüssigkeitsspiegels entweichen kann. Dadurch fällt dann die Auftriebskraft weg, so dass die Umwälzeinrichtung unter Zerstörung der Schwimmdecke aufgrund ihres Eigengewichtes in der Flüssigkeit absinkt.

Vorteilhafterweise kann unmittelbar unterhalb der Wandung des oberen Einbaukörpers wenigstens eine, verschliessbare, mit dem Gasraum des Gärkessels in Verbindung bringbare Öffnung vorgesehen sein, wodurch ein rasches und vollkommenes Ablassen des unter dem oberen Einbaukörper angesammelten Gases ermöglicht ist. Dabei kann die Öffnung in dem die Einbaukörper tragenden Rohr vorgesehen sein, in welchem zum Verschliessen der Öffnung oberhalb derselben ein Schieber vorgesehen ist, der durch eine mit einem festen Anschlag in Wirkverbindung bringbare Steuereinrichtung in Offenstellung bzw. in Schliessstellung bringbar ist, wodurch ein gesteuertes Ablassen des Gases erzielt wird. Weiters kann zwischen den beiden Einbaukörpern ein dritter kappenartiger, mit seiner offenen Seite dem unteren Einbaukörper zugewandter Einbaukörper vorgesehen sein, unterhalb dessen Wandung gleichfalls eine Öffnung in dem die Einbaukörper tragenden Rohr vorgesehen ist, welche mit der Öffnung unterhalb der Wandung des oberen Einbaukörpers in Verbindung steht. Das mit dem dritten Einbaukörper eingefangene Gas wird damit unter den oberen Einbaukörper geleitet, wodurch ein Verlegen des oberen Schirmes mit festem Schwimmdeckenmaterial verhindert ist. Weiters kann oberhalb der Wandung des unteren Einbaukörpers in dem die Einbaukörper tragenden, nach unten offenen Rohr ebenfalls eine Öffnung vorgesehen sein, wodurch sich im unteren Einbaukörper absetzendes Sediment beim Aufwärtsbewegen der Umwälzeinrichtung zufolge der dadurch entstehenden Strömung aus dem Einbaukörper herausgeschwemmt wird, so dass ein Verlegen desselben mit Sediment verhindert ist.

Der Erfindungsgegenstand wird nachstehend an Hand der Zeichnung näher erläutert. Es zeigt

Fig. 1 ein Diagramm betreffend den Zusammenhang zwischen eingesetztem $H_2$ und produziertem $CH_4$,

Fig. 2 im Vertikalschnitt ein Ausführungsbeispiel des Gärkessels bei in oberer Endstellung befindlicher Umwälzeinrichtung und

Fig. 3 schematisch den Gesamtaufbau eines Ausführungsbeispiels der erfindungsgemässen Anlage.

Zu dem erfindungsgemässen Verfahren ist dabei noch folgendes zu bemerken:

Die übliche Umsetzung von Kohlenstoff zu Methan erfolgt nach der Bruttogleichung

$$C + 2H_2 \rightarrow CH_4,$$

wobei diese Umsetzung über zwei Stufen erfolgt, nämlich

$$C + 2O_{nasc.} \rightarrow CO_2$$
$$CO_2 + 2H_2 \rightleftharpoons CH_4 + 2O_{nasc.}$$

Es fungiert dabei also der nascierende Sauerstoff als «Katalysator», der den Kohlenstoff aufnimmt und als $CO_2$ mit $2H_2$ zu $CH_4$ vereinigt, bei welcher Reaktion wieder $2O_{nasc.}$ freiwerden. Daraus ist ersichtlich, dass bei Vorliegen einer genügenden Menge an leicht aufschliessbarem Kohlenstoff die gebildete Methanmenge nur von der Menge des zur Verfügung stehenden Wasserstoffs abhängt. Leicht aufschliessbarer Kohlenstoff ist dabei jeder ungesättigte und/oder nichtaromatische Kohlenstoff. In der Praxis finden organische Abfallprodukte der Landwirtschaft als derartige Kohlenstoffquelle Anwendung.

Die angeführten Reaktionsverhältnisse werden auch durch die folgende Tabelle bestätigt, deren Werte nach dem Molenbruch

$$X_1 = \frac{n_1}{\sum_i n_1}$$

berechnet sind, in welchem bedeutet:

$X_1$: Molenbruch der Substanz 1

$n_1$: Molzahl der Substanz 1

$\sum_i n_1$ : Summe aller Molzahlen der im Gemisch enthaltenen Substanzen $n_1$,

| Edukte (Ausgangsstoffe) n.$CO_2$ : n.$H_2$ | $X_{H_2}$ | Produkte n.$CO_2$ : n.$CH_4$ | $X_{CH_4}$ |
|---|---|---|---|
| 1 : 2,0 | 0,667 | 1 : 1,0 | 0,500 |
| 1 : 2,2 | 0,686 | 1 : 1,1 | 0,524 |
| 1 : 2,4 | 0,706 | 1 : 1,2 | 0,546 |
| 1 : 2,6 | 0,722 | 1 : 1,3 | 0,565 |
| 1 : 2,8 | 0,737 | 1 : 1,4 | 0,583 |
| 1 : 3,0 | 0,750 | 1 : 1,5 | 0,600 |
| 1 : 4,0 | 0,800 | 1 : 2,0 | 0,667 |
| 1 : 5,0 | 0,830 | 1 : 2,5 | 0,714 |
| 1 : 6,0 | 0,875 | 1 : 3,0 | 0,750 |
| 1 : 10,0 | 0,909 | 1 : 5,0 | 0,833 |

In Fig. 1 sind diese Werte graphisch wiedergegeben, wobei sich aus diesem Diagramm der direkte Zusammenhang zwischen der Konzentration von $H_2$ im Eduktgas (Ausgangsgas) und der Konzentration von $CH_4$ im Produktgas besonders deutlich ergibt.

Würde man also in einen, grosse Mengen an leicht aufschliessbarem Kohlenstoff enthaltenden Reaktor $CO_2$ einblasen, so dass gegenüber dem vorhandenen Wasserstoff ein Kohlenstoffüberschuss besteht, dass also das oben angeführte Verhältnis von $CO_2$:$H_2$ von 1:4 zugunsten des $CO_2$ verschoben ist, dann würde es lediglich zu einer «Verdünnung» des gebildeten Methans durch das $CO_2$ kommen. Anderseits stellt auch $CO_2$ einen leicht aufschliessbaren Kohlenstoff dar (siehe die zweite Teilreaktion der Methanumsetzung), welcher dann, wenn keine andere leicht aufschliessbare Kohlenstoffquelle vorliegt, in Methan umgewandelt wird, wobei dann bei Einbringen von gasförmigem $CO_2$ das Verhältnis von $CO_2$:$H_2$ von 1:4 und bei Einbringen eines Gemisches von $CO_2$ und CO ein Verhältnis von $CO_2$:$H_2$ von 1:2:10 günstig ist, um eine möglichst vollständige Umsetzung des vorhandenen Kohlenstoffs zu erzielen.

Mit dem erfindungsgemässen Verfahren können auch Saccharide, Alkohole, Aceton, organische Säuren, Harnstoff, u.dgl. in Methan umgesetzt werden, wenn sie entsprechend konditioniert an der geeigneten Stelle in die Methangärung eingeführt werden. Die Abbaugeschwindigkeit für die Materialien ist dabei von der Bakterienpopulation und der Art der, in einer für diese eingeführten Materialien in geeigneter Weise assimilierten Bakterienstämme in der Stammgärung abhängig.

Beispiel

In einem mehrstufigen Biogasreaktor werden eine oder mehrere Vorgärstufen, eine Hauptgärstufe, also eine Methangärung, und wenigstens eine Nachgärstufe voneinander getrennt geführt. Die Vorgärstufen dienen dabei im wesentlichen dazu, um das Gärmaterial aerob aufzuschliessen und dann den enthaltenen freien Sauerstoff zu verarbeiten, da die Methanbakterien sehr anfällig gegen freien Sauerstoff sind und durch denselben leicht abgetötet werden können.

Wird in eine in voller Gärung befindliche Biomethangärung eine Nachfüllcharge, wie z.B. Stallmist, Grünpflanzen, organische Abfälle aus Müll od.dgl., eingeführt, so wird dieses Material zunächst in einer Zerkleinerungsmaschine zermahlen, wobei dieses Material gleichzeitig mit Wasser auf 10% Trockensubstanz verdünnt und während des Mahlvorganges zwangsläufig mit feinblasig eingebrachter Luft, u.zw. in einer Menge von 20 l pro $m^3$ Gärmaterial und Stunde Verweilzeit, angereichert wird. Diese Mischung wird nun in ein Vorgärbecken eingebracht, wobei die Verzweilzeit in dieser ersten Vorgärstufe, je nach dem artspezifischen Gärverhalten des verwendeten Materials, etwa 2–48 Stunden beträgt. Sobald der gesamte freie Sauerstoff verbraucht ist, wird das fliessfähige aufgeschlämmte, noch aerob gärende Material in einen Vorgärkessel, in welchem anaerobe Bedingungen herrschen, eingeleitet, wobei das sich in dieser zweiten Vorgärstufe bildende Gas, sowie auch aus anderen Quellen stammende, keinen freien Sauerstoff enthaltende, zur Umsetzung in Methan geeignete Gase, die sich vorwiegend aus CO, $CO_2$, $H_2$, $H_2S$ u.a. zusammensetzen, wie z.B. Pyrolysegas, Raffinerieabgase u.dgl., in das sich in der eigentlichen Methangärung befindliche Material eingeblasen werden.

Die Verweilzeit in der eigentlichen Methangärstufe beträgt, je nach Art des verwendeten Materials, etwa 20–200 Stunden.

In einer ersten Nachgärstufe wird aus dem von der eigentlichen Methangärung stammenden Material das noch vorhandene unverarbeitete Gas ausgetrieben und in die zweite Vorgärstufe rückgeführt, wo es für die Verarbeitung in der eigentlichen Methangärung aufbereitet wird. Das abgearbeitete Material wird anschliessend in einer weiteren Nachgärstufe analog wie in der ersten Vorgärstufe belüftet und nochmals in eine aerobe Rotte übergeführt, um jene gärfähigen Materialien, die während des Durchgangs durch die vorhergehenden Prozessstufen infolge der kurzen Verweilzeit nicht abgebaut wurden, das sind z. B. in Lignin- oder andere Mantelstrukturen eingeschlossene Materialien, doch noch zur Energiegewinnung nutzbar zu machen.

Dieser Nachgärprozess dauert 10–60 Tage. Das sich dabei bildende Gas wird in die erste Vorgärstufe rückgeführt, um einen eventuell vorhandenen Rest an freiem Sauerstoff zu verarbeiten.

Die in diesem Gärprozess entstehende Prozesswärme steht als Bestandteil des Gesamtenergieertrages zur wirtschaftlichen Nutzung zur Verfügung.

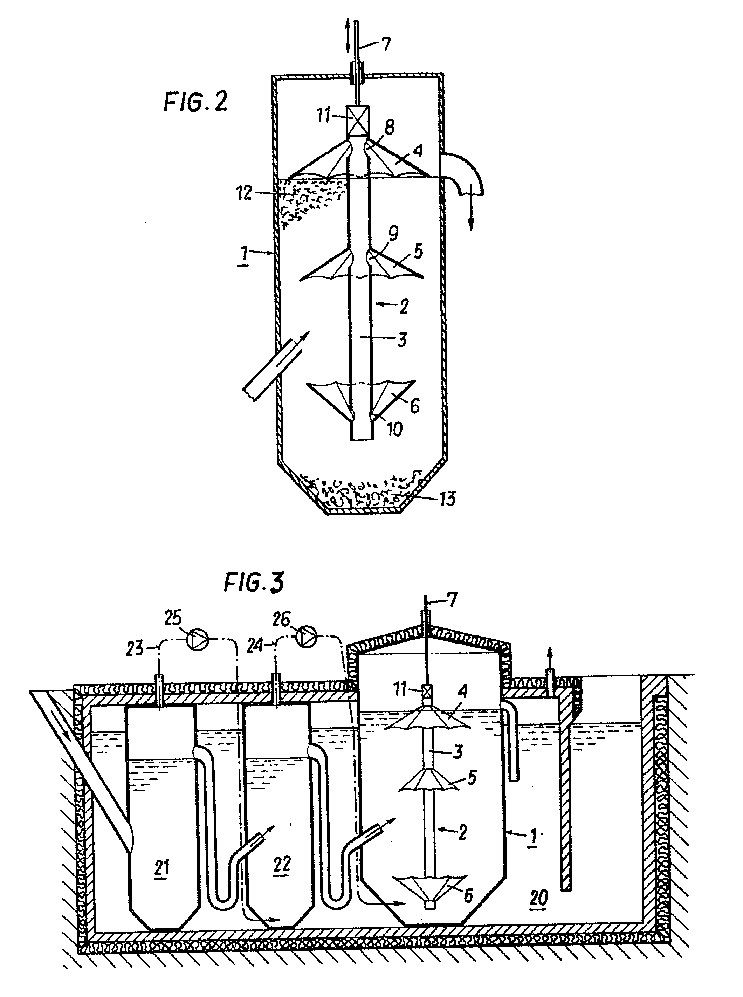

Bei der in Fig. 2 und Fig. 3 dargestellten Anlage ist mit 1 ein Gärkessel bezeichnet, in welchem eine Umwälzeinrichtung 2 vorgesehen ist. Diese Umwälzeinrichtung besteht aus einem oben und unten offenen Rohr 3, an welchem schirmförmige Einbaukörper 4, 5, 6 angeordnet sind, wobei der oberste Einbaukörper 4 und der unterste Einbaukörper 6 mit ihren offenen Seiten zueinanderweisen. Der mittlere Einbaukörper 5 weist mit seiner offenen Seite ebenfalls zum unteren Einsatzkörper 6. Das die Einbaukörper 4, 5, 6 tragende Rohr 3 ist dabei an einer zentralen vertikalen Achse 7 in seiner Längsrichtung verschiebbar geführt. An dem die Einbaukörper 4, 5, 6 tragenden Rohr sind knapp unterhalb der Wandungen des oberen Einbaukörpers 4 und des mittleren Einbaukörpers 5 sowie knapp oberhalb der Wandung des untersten Einbaukörpers 6 Öffnungen 8, 9, 10 vorgesehen. Das obere Ende des Rohres 3 ist mittels eines Schiebers 11 verschliessbar, der durch eine herkömmliche, nicht dargestellte, mit einem Anschlag in Wirkverbindung bringbare Steuereinrichtung in die Offenstellung bzw. in die Schliessstellung bringbar ist. Mit 12 ist die Schwimmdecke und mit 13 das Sediment im Gärkessel 1 bezeichnet.

Bei Betrieb der Anlage entsteht im Gärkessel 1 Methangas, das in Form von Blasen im Gärsubstrat aufsteigt. Dabei sammelt sich das aufsteigende Gas unter dem oberen und dem mittleren Einbaukörper 4 bzw. 5. Das unter dem mittleren Einbaukörper 5 gesammelte Gas tritt durch die Öffnungen 9 in das Rohr 1 ein, steigt in diesem nach oben und tritt durch die Öffnungen 8 unter dem oberen Einbaukörper 4 aus. Das unter dem oberen Einbaukörper 4 befindliche Gas kann zufolge des geschlossenen Schiebers 1 nicht aufsteigen und bildet eine Gasblase. Durch diese Gasansammlung unter dem oberen Einbaukörper 4 wird der Auftrieb der Umwälzeinrichtung 2 so gross, dass letztere entlang der zentralen Achse 7 aufsteigt. Sobald sich die Umwälzeinrichtung 2 in ihrer oberen Endstellung befindet, bei welcher der oberste Einbaukörper 4 oberhalb der Schwimmdecke 12 liegt, wird der Schieber 11 geöffnet, wodurch das unter dem obersten Einbaukörper 4 befindliche Gas durch das Rohr 1 hindurch in den Gasraum des Gärkessels 1 entweichen kann. Damit fehlt dann der den Auftrieb bewirkende Gaspolster unter dem obersten Einbaukörper 4, wodurch die Umwälzeinrichtung 2 im Gärkessel 1 absinkt, wobei der oberste Einbaukörper 4 die Bestandteile der Schwimmdecke 12 nach unten mitnimmt und in der gärenden Flüssigkeit verteilt. Durch die durch den unteren Einbaukörper 6 im unteren Teil des Gärkessels 1 erzeugten Turbulenzen wird das Sediment 13 aufgewirbelt, wobei sich im untersten Einbaukörper 6 ansammelndes Sediment durch die Öffnungen 10 und durch den unteren Teil des Rohres 3 hindurch ausgeschwemmt wird.

Wie aus Fig. 3 ersichtlich, ist der Gärkessel 2 in einem geschlossenen, isolierten Wasserbecken 20 angeordnet, in welchem sich auch ein Angärkessel 21 und ein Vorgärkessel 22 befinden. Das Wasserbecken 20 dient dabei als Nachgärbecken, wobei die bei der Nachgärung entstehende Prozesswärme der Vorgärung und der Methangärung direkt zugeführt wird, da sich die entsprechenden Gärkessel direkt in dem Nachgärbecken 20 befinden, so dass also das Nachgärbecken als Wärmetauscher fungiert. Mit 23 und 24 sind Gasübertrittsleitungen bezeichnet, in welche Hilfskompressoren 25, 26 zur Überwindung des Flüssigkeitsgegendruckes eingebaut sind.

**Patentansprüche**

1. Anlage zur Herstellung von Biogas, in welcher organische Stoffe mit Abfallmaterialien, insbesondere aus landwirtschaftlichen Betrieben, in mehreren Stufen zu Methangas vergoren werden, mit einem Behälter (21) für die aerobe Angärung und mehreren Gärkesseln (1) wobei die Gärkessel (1) über eine Einrichtung (2) zur Umwälzung des Gärmaterials verfügen, dadurch gekennzeichnet, dass die Einrichtung (2) zur Umwälzung des Gärmaterials durch wenigstens zwei, kappenartige, vorzugsweise schirmförmige, Einbaukörper (4, 6) gebildet ist, welche mit ihren offenen Seiten zueinander an einem Rohr (3) angebracht sind, welches an einer zentralen Achse (7) in einer Längsrichtung verschiebbar geführt ist.

2. Anlage nach Anspruch 1, dadurch gekennzeichnet, dass unmittelbar unterhalb der Wandung des oberen Einbaukörpers (4) wenigstens eine verschliessbare, mit dem Gasraum des Gärkessels (1) in Verbindung bringbare Öffnung (8) vorgesehen ist.

3. Anlage nach Anspruch 2, dadurch gekennzeichnet, dass die Öffnung (8) in dem die Einbaukörper (4) tragenden Rohr (3) vorgesehen ist, in welchem zum Verschliessen der Öffnung (8) oberhalb derselben ein Schieber (1) vorgesehen ist, der durch eine mit einem festen Anschlag in Wirkverbindung bringbare Steuereinrichtung in Offenstellung bzw. in Schliessstellung bringbar ist.

4. Anlage nach Anspruch 1 oder 2, dadurch gekennzeichnet, dass zwischen den beiden Einbaukörpern (4, 6) ein dritter kappenartiger, mit seiner offenen Seite dem unteren Einbaukörper (6) zugewandter Einbaukörper (5) vorgesehen ist, unterhalb dessen Wandung gleichfalls eine Öffnung (9) in dem die Einbaukörper (4, 5, 6) tragenden Rohr (3) vorgesehen ist, welche mit der Öffnung (8) unterhalb der Wandung des oberen Einbaukörpers (4) in Verbindung steht.

5. Anlage nach einem der Ansprüche 1 bis 4, dadurch gekennzeichnet, dass oberhalb der Wandung des unteren Einbaukörpers (6) in dem die Einbaukörper (4, 5, 6) tragenden, nach unten offenen Rohr (3) ebenfalls eine Öffnung (10) vorgesehen ist.

**Claims**

1. Installation for the production of biogas in which organic substances together with waste materials, particularly from agricultural operations, are fermented into methane in a number of steps, including a container (21) for the initial aerobic fermentation and a plurality of fermentation tanks (1), the fermentation tanks (1) having a device (2) for circulated the fermenting material, characterised in that the device (2) for circulating the fermenting material is constituted by at least two cap-like, preferably umbrella-shaped bodies (4, 6) which are mounted with their open sides directed towards one another on a tube (3) which is guided on a central spindle (7) so as to be displaceable in a longitudinal direction.

2. Installation as claimed in claim 1, characterised in that immediately below the wall of the upper body (4) there is provided at least one closeable opening (8) which may be brought into communication with the gas space of the fermentation tank (1).

3. Installation as claimed in claim 2, characterised in that the opening (8) is provided in the tube (3) carrying the bodies (4), in which tube there is provided a slider (11) above the opening (8) for closing the same, which slider may be moved into an open position or a closed position by a control device which can be operatively connected to a fixed abutment.

4. Installation as claimed in claim 1 or 2, characterised in that provided between the two bodies (4, 6) there is a third cap-like body (5) whose open side is directed towards the lower body (6) and below the wall of which there is also provided an opening (9) in the tube (3) carrying the bodies (4, 5, 6), which opening is in communication with the opening (8) below the wall of the upper body (4).

5. Installation as claimed in one of claims 1 to 4, characterised in that an opening (10) is also provided above the wall of the lower body (6) in the downwardly open tube (3) carrying the bodies (4, 5, 6).

**Revendications**

1. Installation pour produire du gaz biologique (de fermentation), dans laquelle on fait fermenter des matières organiques avec des déchets, en particulier provenant d'exploitation agricoles, en plusieurs stades, pour obtenir du gaz méthane, comportant un récipient (21) pour la fermentation aérobie et plusieurs cuves de fermentation (1), les cuves de fermentation (1) disposant d'un agencement (2) pour brasser la matière de fermentation, caractérisée en ce que l'agencement (2) pour brasser la matière de fermentation est formé par au moins deux pièces d'insertion (4, 6) en forme de capuchon, de préférence en forme de parapluie, qui sont placés avec leurs côtés ouverts en regard sur un tube (3) qui est guidé de façon à pouvoir se déplacer en direction longitudinale sur un axe central (7).

2. Installation selon la revendication 1, caractérisée en ce qu'on prévoit juste au-dessous de la paroi de la pièce d'insertion supérieure (4) au moins une ouverture (8) pouvant être fermée, qui peut être mise en communication avec la chambre du gaz de la cuve de fermentation (1).

3. Installation selon la revendication 2, caractérisée en ce que l'ouverture (8) est prévue dans le tube (3) supportant les pièces d'insertion (4) dans lequel est prévu, pour fermer l'ouverture (8), audessus de celle-ci, un coulisseau (11) qui peut être amené en position d'ouverture ou en position de fermeture par un mécanisme de commande pouvant être mis en interaction avec une butée fixe.

4. Installation selon la revendication 1 ou 2, caractérisée en ce qu'il est prévu entre les deux pièces d'insertion (4, 6) une troisième pièce d'insertion (5) en forme de capuchon, dont le côté ouvert est situé vers la pièce d'insertion inférieure (6), au-dessous de la paroi de laquelle est également prévue une ouverture (9) dans le tube (3) supportant les pièces d'insertion (4, 5, 6), laquelle est en communication avec l'ouverture (8) située au-dessous de la paroi de la pièce d'insertion supérieure (4).

5. Installation selon l'une des revendications 1 à 4, caractérisée en ce qu'on prévoit également au-dessus de la paroi de la pièce d'insertion inférieure (6), dans le tube (3) ouvert vers le bas, supportant les pièces d'insertion (4, 5, 6), une ouverture (10).

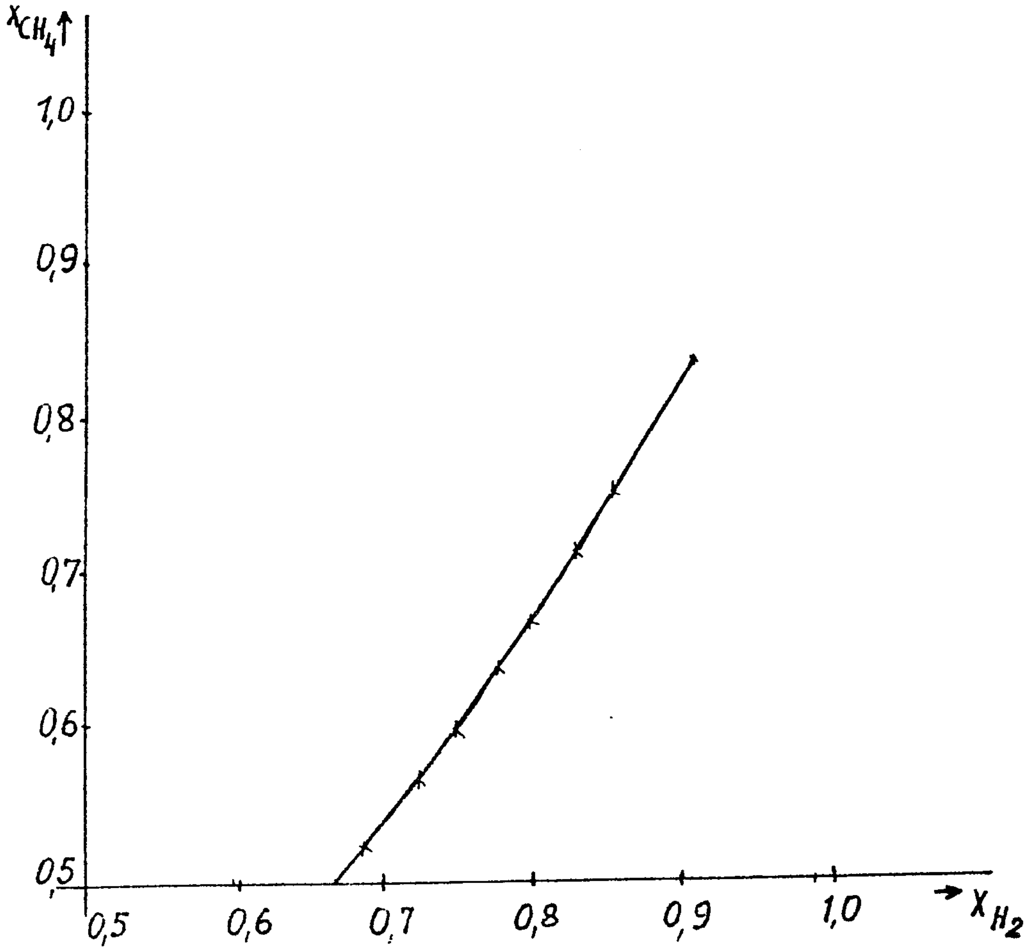


FIG. 1

FIG. 2

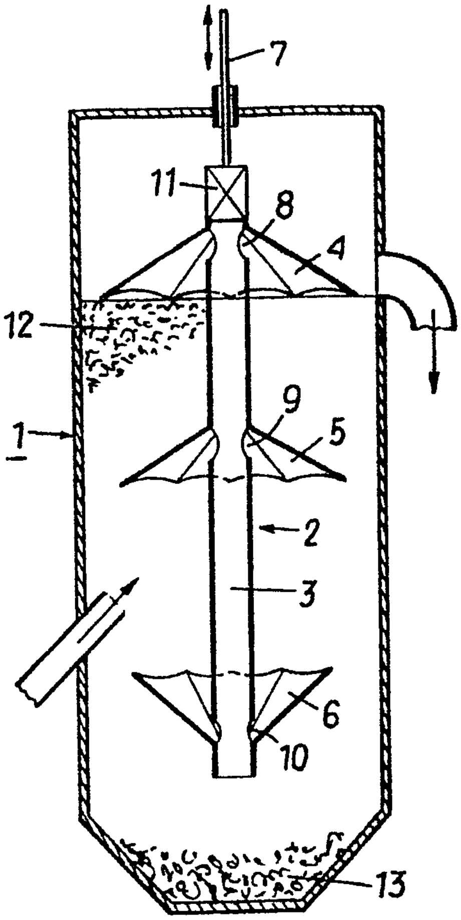


FIG. 3